# FASCICULE DE BREVET EUROPEEN

(11) **EP 3 322 990 B1**
(45) Date de publication et mention de la délivrance du brevet: **22.05.2019**
(21) Numéro de dépôt: 16750957.9
(22) Date de dépôt: 13.07.2016
(51) Int. Cl.: G01N 33/68, G01N 1/28

(54) **ANALYSE PROTÉOMIQUE DE COMPARTIMENTS SUBCELLULAIRES**
PROTEOMANALYSE VON SUBZELLULÄREN KOMPARTIMENTEN
PROTEOMIC ANALYSIS OF SUBCELLULAR COMPARTMENTS

(30) Priorité: 15.07.2015 FR 1501491
(43) Date de publication de la demande: 23.05.2018
(73) Titulaire: Université de Bordeaux, 33000 Bordeaux (FR); INSERM - Institut National de la Santé et de la Recherche Médicale, 75013 Paris (FR)
(72) Inventeur: SALTEL, Frédéric, 33400 Talence (FR); RAYMOND, Anne-Aurélie, 33700 Mérignac (FR); EZZOUKHRY, Zakaria, F-80000 Amiens (FR)
(74) Mandataire: Novagraaf Technologies
(86) Numéro de dépôt international: PCT/FR2016/051808
(87) Numéro de publication internationale: WO 2017/009579

(56) Documents cités:
- US-A1- 2011 215 233
- JAMES B. LEVERENZ ET AL: "Proteomic Identification of Novel Proteins in Cortical Lewy Bodies", BRAIN PATHOLOGY., vol. 17, no. 2, 1 avril 2007 (2007-04-01), pages 139-145, XP055264220, CH ISSN: 1015-6305, DOI: 10.1111/j.1750-3639.2007.00048.x
- ONG SHAO-EN ET AL: "Mass spectrometry-based proteomics turns quantitative", NATURE CHEMICAL BIOLOGY, NATURE PUBLISHING GROUP, GB, vol. 1, no. 5, 1 octobre 2005 (2005-10-01) , pages 252-262, XP002430016, ISSN: 1552-4450, DOI: 10.1038/NCHEMBIO736
- ZANG LI ET AL: "Proteomic analysis of ductal carcinoma of the breast using laser capture microdissection, LC-MS, and 16O/18O isotopic labeling", JOURNAL OF PROTEOME RESEARCH, ACS, WASHINGTON, DC, US, vol. 3, no. 3, 1 mai 2004 (2004-05-01), pages 604-612, XP002345280, ISSN: 1535-3893, DOI: 10.1021/PR034131L
- SETHI SUMIT ET AL: "Approaches for targeted proteomics and its potential applications in neuroscience", JOURNAL OF BIOSCIENCES, INDIAN ACADEMY OF SCIENCES, IN, vol. 40, no. 3, 8 juillet 2015 (2015-07-08), pages 607-627, XP035534304, ISSN: 0250-5991, DOI: 10.1007/S12038-015-9537-1 [extrait le 2015-07-08]
- BAOGANG J. XU: "Combining laser capture microdissection and proteomics: Methodologies and clinical applications", PROTEOMICS - CLINICAL APPLICATIONS, vol. 4, no. 2, 1 février 2010 (2010-02-01) , pages 116-123, XP055264287, DE ISSN: 1862-8346, DOI: 10.1002/prca.200900138
- SERHIY HAVRYLOV ET AL: "MS/MS-based strategies for proteomic profiling of invasive cell structures", PROTEOMICS, vol. 15, no. 2-3, 15 décembre 2014 (2014-12-15), pages 272-286, XP055264372, DE ISSN: 1615-9853, DOI: 10.1002/pmic.201400220
- PASQUALE CERVERO ET AL: "Proteomic analysis of podosome fractions from macrophages reveals similarities to spreading initiation centres", EUROPEAN JOURNAL OF CELL BIOLOGY, vol. 91, no. 11-12, 1 novembre 2012 (2012-11-01), pages 908-922, XP055264507, DE ISSN: 0171-9335, DOI: 10.1016/j.ejcb.2012.05.005
- CHAD P. SATORI ET AL: "Review on recent advances in the analysis of isolated organelles", ANALYTICA CHIMICA ACTA, vol. 753, 1 novembre 2012 (2012-11-01), pages 8-18, XP055264591, NL ISSN: 0003-2670, DOI: 10.1016/j.aca.2012.09.041
- Anne-Aurélie Raymond: "A combined laser microdissection and mass spectrometry method for proteomic analysis of tissue sections for the identification of tumor signatures - Session 4 : Clinical and biological samples: Opportunies and challenges for translational research", , 1 novembre 2015 (2015-11-01), XP055263371, Extrait de l'Internet: URL:https://siric-brio.com/wp-content/uplo ads/2015/11/BRIO2015-session4-Anne-aurélie -Raymond.pdf [extrait le 2016-04-06]

## Description

### Domaine technique

La présente invention se rapporte à un nouveau procédé d'analyse protéomique de compartiments subcellulaires combinant une microdissection laser suivie d'une analyse par spectrométrie de masse.

La présente invention trouve notamment une application dans le domaine de la recherche, mais également dans le domaine du diagnostic médical.

Dans la description ci-dessous, les références entre crochets (**[ ]**) renvoient à la liste des références présentée à la fin du texte.

### Etat de la technique

L'analyse par spectrométrie de masse permet d'identifier et de quantifier les protéines d'un échantillon biologique. Cette analyse peut être réalisée à partir de tissus entiers ou d'extraits cellulaires et après séparation biochimique de compartiments cellulaires dans le cadre d'une analyse protéomique subcellulaire. Cependant, la spécificité et la reproductibilité des méthodes de fractionnement subcellulaire sont largement discutées du fait des biais induits par la lyse cellulaire qui entraîne une déstabilisation des compartiments cellulaires et des complexes moléculaires qui les composent [1, 2].

Aujourd'hui, l'imagerie MALDI (Matrix-Assisted Laser Desorption/lonization Imaging Mass Spectrometry ou MALDI-IMS) apporte une nouvelle dimension aux analyses histologiques car elle combine sensibilité et sélectivité et permet de visualiser directement l'arrangement des biomolécules dans un tissu. Toutefois cette nouvelle technologie présente pour le moment deux limites importantes (i) une résolution latérale dans le meilleur des cas de 20 µm incompatible avec l'analyse des compartiments subcellulaires [3] et (ii) contrairement à l'approche bottom-up basée sur la digestion des protéines, l'imagerie MALDI nécessite une approche top-down qui repose sur l'analyse de protéines entières et qui en est encore aujourd'hui à ses balbutiements [4-6]. Gregorich et al. ([4]) soulignent bien les obstacles à la mise en oeuvre de cette technologie en termes de solubilité des protéines, séparation et détection des protéines de grande taille, associés à une baisse de rendement et une absence d'automatisation. Dans le cadre de leurs travaux utilisant le couplage imagerie MALDI et l'identification Top Down en spectrométrie de masse, Ye et al. ([5]) pointent les limites de cette technique d'imagerie, qui nécessite de travailler sur coupes congelées et ne permet pas le travail sur coupes fixées, et dont la résolution limitée ne permet pas une analyse subcellulaire. Enfin, Ait-Belkacem et al. ([6]), qui utilisent le couplage imagerie MALDI et l'identification Top Down en spectrométrie de masse pour caractériser les structures moléculaires des glioblastomes, n'obtiennent dans ce cas qu'une résolution spatiale de 30µm, ce qui ne permet pas d'analyse à l'échelle subcellulaire.

Le document EP1601450 ([9]), qui décrit une méthode de détection d'analytes dans un lysat, ne permet pas non plus une analyse à une échelle subcellulaire.

Leverenz et al. ([10]) décrivent une méthode d'analyse protéomique subcellulaire d'un échantillon de cerveau comprenant une étape de microdissection laser de corps de Lewy suivi d'une étape d'analyse par spectrométrie de masse. Toutefois le niveau de résolution n'est pas satisfaisant pour une réelle analyse à l'échelle subcellulaire.

Le document US 2011/0215233 ([11]) décrit un procédé d'analyse subcellulaire par ablation laser et spectrométrie de masse. Toutefois, même si une séparation noyau/cytoplasme est réalisée et que les auteurs mentionnent la possibilité d'appliquer leur procédé à des organites cellulaires, aucun exemple n'est fourni à ce niveau de résolution. Aussi le niveau de résolution ne parait pas assez satisfaisant pour une réelle analyse à l'échelle subcellulaire.

Il existe donc un réel besoin d'une approche alternative palliant ces défauts, inconvénients et obstacles de l'art antérieur, en particulier d'un procédé permettant d'obtenir une meilleure résolution, mais également d'améliorer la spécificité et la reproductibilité des analyses.

### Description de l'invention

Pour ce faire les Inventeurs ont mis au point une nouvelle approche qui représente une alternative à plusieurs méthodologies comme la compartimentation cellulaire par centrifugation et dans certains cas l'immunoprécipitation. De plus, compte-tenu des avancées actuelles et des limitations spatiales et d'identification, cette nouvelle approche propose une alternative à l'imagerie MALDI.

La présente invention consiste essentiellement en un procédé permettant l'identification et la quantification des protéines présentes dans un compartiment cellulaire (réticulum endoplasmique, centriole, appareil de golgi, synapses, etc...). Cette analyse n'est pas réalisable avec les techniques classiques (spectrométrie de masse, MALDI-IMS) en raison de la non-spécificité et de la non-reproductibilité des méthodes de fractionnements subcellulaires d'une part et de la forte contamination par les éléments extérieurs (air ambiant, solutions, etc...) d'autre part, et également de la limite technique de ces approches à l'échelle micrométrique.

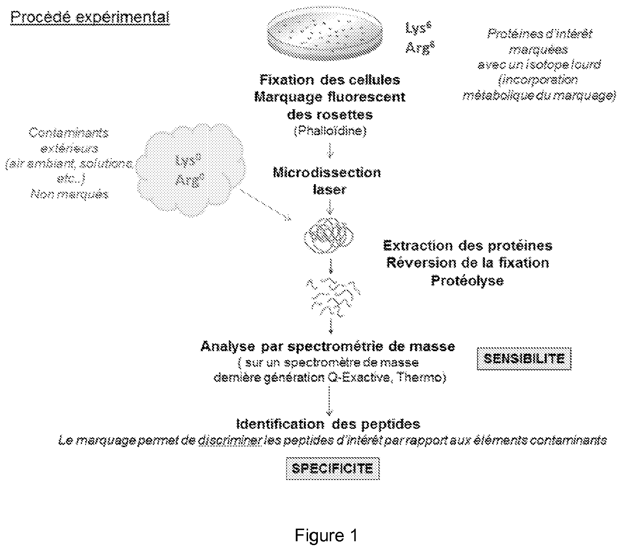

Le procédé de l'invention combine une étape de marquage isotopique d'un échantillon biologique test (*e.g.* une culture cellulaire), une étape de microdissection laser pour la découpe sélective d'éléments subcellulaires et une analyse protéomique par spectrométrie de masse (cf. figure 1).

Cette nouvelle approche applicable sur des cultures cellulaires humaines ou animales ou sur des coupes tissulaires animales, combine la microdissection laser qui permet une découpe sélective des éléments subcellulaires (à une résolution de 0,6 µm) et l'analyse par spectrométrie de masse. Cette nouvelle approche permet ainsi de se placer dans une résolution spatiale compatible avec la séparation de compartiments subcellulaires tels que le noyau, la membrane cytoplasmique, la membrane nucléaire, les vésicules, la mitochondrie, le lysosome, le centriole, le protéasome, les adhésions focales, le lamellipode, les filopodes, la rosette d'invadosomes, le réticulum endoplasmique, l'appareil de golgi. Il est également possible de découper des compartiments extracellulaires présents entre deux cellules, comme les jonctions cellules-cellules (*e.g.* les synapses). En outre cette nouvelle combinaison originale permet d'améliorer la spécificité et la reproductibilité des analyses par rapport aux méthodes actuellement pratiquées de fractionnement subcellulaire suivi d'une analyse par spectrométrie de masse.

Cette nouvelle approche intéresse en premier lieu les laboratoires de recherche, qui pour beaucoup recherchent de nouvelles méthodes pour identifier de nouveaux partenaires au sein des organelles ou structures subcellulaires. En outre, cette nouvelle approche permet de comparer la composition protéique d'un compartiment subcellulaire entre différents échantillons cellulaires, par exemple celle d'un compartiment subcellulaire issu d'une cellule normale et celle d'un compartiment subcellulaire issu d'une cellule tumorale. Cette nouvelle approche permet également d'identifier des protéines surexprimées dans le cadre de différentes pathologies. Enfin le fait de pouvoir cibler un compartiment subcellulaire permet d'identifier et d'analyser des protéines qui avec une approche non ciblée seraient minoritaires et non détectables dans une gamme protéique beaucoup plus complexe.

La présente invention a donc pour objet un procédé d'analyse protéomique subcellulaire d'un échantillon biologique test comprenant les étapes suivantes :
a) Marquage isotopique métabolique des protéines d'un échantillon biologique test;
b) Fixation dudit échantillon;
c) Marquage du compartiment subcellulaire test;
d) Microdissection laser dudit compartiment subcellulaire;
e) Extraction des protéines dudit compartiment subcellulaire, réversion de la fixation et protéolyse;
f) Analyse des peptides issus de l'étape e) par spectrométrie de masse;
g) Identification des peptides analysés.

On entend par « échantillon biologique test », par exemple un échantillon de cellules humaines ou animales ou un échantillon issu d'une coupe de tissu d'un animal dont on veut connaitre la composition qualitative et/ou quantitative en protéine(s) d'au moins un compartiment subcellulaire.

On entend par « éléments constitutifs du compartiment subcellulaire », toute molécule ou protéine présente au sein d'un compartiment microdisséqué. A titre d'exemple, l'actine filamenteuse est un élément constitutif des rosettes d'invadosomes, l'ADN est un élément constitutif du noyau, etc....

Selon la présente invention, le procédé d'analyse protéomique comprend, préalablement à l'étape de microdissection, une étape de marquage isotopique métabolique des protéines de l'échantillon cellulaire test, une étape de fixation des cellules (*e.g.* afin de limiter les contaminants en Z sur des expériences sur cellules entières, il est possible au préalable de fixer, et inclure les cellules en paraffine et réaliser des coupes de 3 µm) dudit échantillon et une étape de marquage du compartiment subcellulaire test.

Selon la présente invention, ledit procédé d'analyse protéomique comprend, postérieurement à l'étape de microdissection laser et préalablement à l'étape d'analyse par spectrométrie de masse, une étape d'extraction des protéines d'intérêt du compartiment subcellulaire, de réversion de la fixation et de protéolyse.

Selon la présente invention, le procédé d'analyse protéomique subcellulaire comprend ou consiste en les étapes suivantes :
a) Marquage isotopique métabolique des protéines d'un échantillon biologique test ;
b) Fixation dudit échantillon ;
c) Marquage du compartiment subcellulaire test ;
d) Microdissection laser dudit compartiment subcellulaire ;
e) Extraction des protéines dudit compartiment subcellulaire, réversion de la fixation et protéolyse ;
f) Analyse des peptides issus de l'étape e) par spectrométrie de masse ;
g) Identification des peptides analysés.

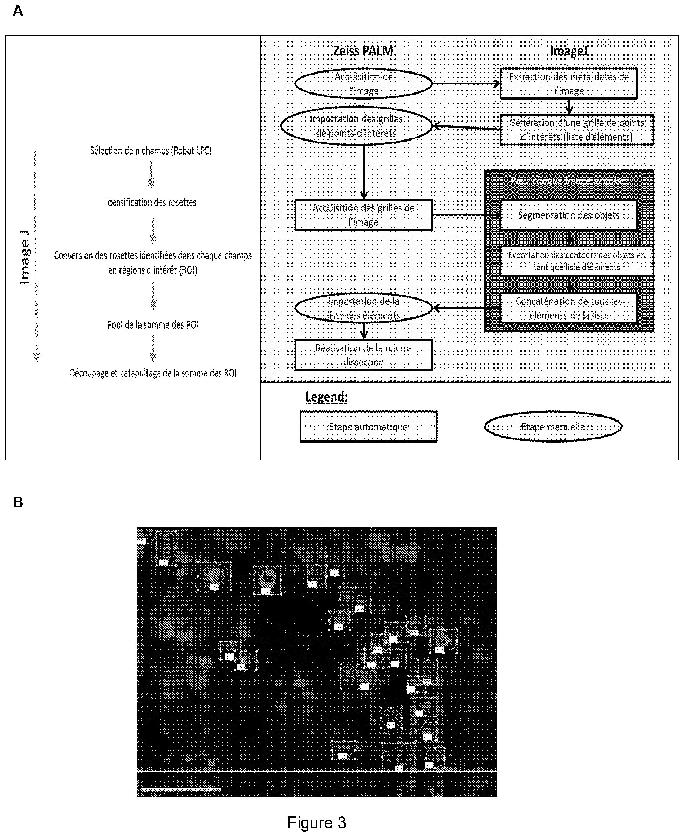

L'étape a) de marquage isotopique peut être réalisée par toute technique de marquage connue de l'homme du métier. Il est à noter que le marquage isotopique métabolique, du style SILAC, n'est pas réalisé lorsque l'échantillon biologique test est une coupe tissulaire déjà marquée. Dans ce cas, le procédé, et notamment l'étape de microdissection laser, peut être automatisée (cf. figure 3).

La mise en oeuvre d'au moins une partie des étapes du procédé peut être avantageusement automatisée. Il peut s'agir notamment de l'étape de microdissection laser. En particulier, dans le cas où le procédé ne comprend pas l'étape de marquage isotopique, cette automatisation concerne l'étape de microdissection laser. Cette automatisation permet avantageusement d'obtenir une quantité suffisante de matériel pour l'analyse en spectrométrie de masse et l'identification des peptides.

Selon un mode de réalisation particulier de la présente invention, l'étape de fixation est réalisée avec un agent réticulant, par exemple le paraformaldéhyde, la formaline, le glutaraldéhyde.

Selon un mode de réalisation particulier de la présente invention, le compartiment subcellulaire est choisi dans le groupe constitué par le noyau, la membrane cytoplasmique, la membrane nucléaire, les vésicules, la mitochondrie, le lysosome, le centriole, le protéasome, les adhésions focales, le lamellipode, les filopodes, la rosette d'invadosomes, le réticulum endoplasmique, l'appareil de golgi, les jonctions cellule-cellule (*e.g.* synapses).

L'étape c) de marquage donc de repérage du compartiment subcellulaire d'intérêt peut être réalisée par différents peptides ou drogues. Selon un mode de réalisation particulier de la présente invention, l'étape de marquage du compartiment subcellulaire est réalisée avec un marqueur fluorescent. Ce marquage peut être réalisé après l'étape de fixation, ou avant par expression constitutive dans le compartiment subcellulaire de l'échantillon test d'un marqueur, typiquement fluorescent. Par exemple, il s'agit du peptide Lifeact ou de la phalloïdine pour l'actine filamenteuse des rosettes d'invadosomes, mais aussi du DAPI comme intercalant de l'ADN qui permet de visualiser les noyaux, de l'ER tracker pour le réticulum endoplasmique, etc.... De manière alternative ou complémentaire, ce marqueur peut être exprimé par les cellules dans lesquelles la microdissection laser d'un compartiment subcellulaire est effectuée.

L'étape e) d'extraction des protéines dudit compartiment subcellulaire test, de réversion de la fixation et/ou de protéolyse peuvent être réalisées par toutes techniques connues de l'homme du métier.

Est aussi décrit un procédé d'identification *in vitro* d'élément constitutif d'un échantillon biologique d'un sujet comprenant une étape de microdissection laser d'un compartiment subcellulaire dudit échantillon suivie d'une étape d'analyse par spectrométrie de masse des éléments constitutifs dudit compartiment subcellulaire, et de comparaison qualitative et/ou quantitative des éléments constitutifs analysés par rapport à un échantillon de référence.

L'élément constitutif peut par exemple être une protéine, notamment une protéine présente dans le complexe d'invasion, dit invadosome. Les invadosomes qui comprennent, les podosomes des cellules normales et les invadopodes des cellules tumorales, forment des complexes protéiques riches en actine spécialisés dans la dégradation protéolytique de la matrice extracellulaire. Cette activité protéolytique confère aux cellules tumorales la capacité de traverser des barrières anatomiques et de migrer à travers les tissus.

Selon un aspect particulier, ledit procédé d'identification *in vitro* comprend en outre préalablement à l'étape de microdissection, au moins une étape choisie parmi le groupe constitué de : marquage isotopique métabolique des protéines de l'échantillon biologique test, fixation dudit échantillon, marquage du compartiment subcellulaire test.

Selon un aspect particulier, ledit procédé d'identification *in vitro* comprend en outre postérieurement à l'étape de microdissection laser et préalablement à l'étape d'analyse par spectrométrie de masse, au moins une étape choisie parmi le groupe constitué de : extraction des protéines d'intérêt du compartiment subcellulaire, réversion de la fixation, protéolyse.

Selon un aspect particulier, le procédé d'identification *in vitro* comprend ou consiste en les étapes suivantes :
a) Fixation dudit échantillon ;
b) Marquage dudit compartiment subcellulaire test ;
c) Microdissection laser dudit compartiment subcellulaire ;
d) Extraction des protéines dudit compartiment subcellulaire, réversion de la fixation et protéolyse ;
e) Analyse des peptides issus de l'étape d) par spectrométrie de masse ;
f) Identification des peptides analysés ;
g) Comparaison qualitative et/ou quantitative des peptides issus de l'étape f) par rapport aux peptides présents dans un échantillon de référence ou à une valeur de référence.

La présente invention a aussi pour objet un procédé d'identification *in vitro* de peptides correspondant à une protéine présente dans un compartiment subcellulaire d'un échantillon biologique d'un sujet comprenant les étapes suivantes:
a) Marquage isotopique métabolique des protéines d'un échantillon biologique d'un sujet;
b) Fixation dudit échantillon;
c) Marquage dudit compartiment subcellulaire test;
d) Microdissection laser dudit compartiment subcellulaire;
e) Extraction des protéines dudit compartiment subcellulaire, réversion de la fixation et protéolyse;
f) Analyse des peptides issus de l'étape e) par spectrométrie de masse;
g) Identification des peptides analysés;
h) Comparaison qualitative et/ou quantitative des peptides issus de l'étape g) par rapport aux peptides présents dans un échantillon de référence ou à une valeur de référence.

Selon un mode de réalisation particulier de la présente invention, le procédé d'identification *in vitro* comprend ou consiste en les étapes suivantes :
a) Marquage isotopique métabolique des protéines d'un échantillon biologique d'un sujet atteint d'une tumeur ;
b) Fixation dudit échantillon ;
c) Marquage dudit compartiment subcellulaire test ;
d) Microdissection laser dudit compartiment subcellulaire ;
e) Extraction des protéines dudit compartiment subcellulaire, réversion de la fixation et protéolyse ;
f) Analyse des peptides issus de l'étape e) par spectrométrie de masse ;
g) Identification des peptides analysés ;
h) Comparaison qualitative et/ou quantitative des peptides issus de l'étape g) par rapport aux peptides présents dans un échantillon de référence ou à une valeur de référence.

Selon un mode de réalisation particulier de l'invention, le compartiment subcellulaire consiste en les rosettes d'invadosomes.

L'étape a) de marquage isotopique peut être réalisée par toute technique de marquage connue de l'homme du métier. Selon un mode de réalisation particulier de la présente invention, l'étape de marquage isotopique est réalisée par la méthode SILAC (Stable Isotope Labelling by Amino acids in Cell culture). Cette méthode SILAC consiste à remplacer un ou plusieurs acide(s) aminé(s) des protéines de l'échantillon test par son équivalent isotopiquement alourdi.

Appliqué à des cellules en culture, le marquage SILAC est un marquage métabolique de cellules cultivées dans un milieu de culture contenant un ou plusieurs acide(s) aminé(s) marqué(s) avec un ou plusieurs isotope(s) lourd(s) (par exemple ¹³C et/ou ¹⁵N). Les acides aminés alourdis sont incorporés au cours des doublements cellulaires pour conduire à un marquage total du protéome cellulaire. La méthode SILAC présente l'avantage d'une incorporation précoce des isotopes sur des cellules vivantes en culture permettant ainsi un marquage homogène des protéines. Cette méthode a été initialement conçue pour pouvoir mélanger selon un ratio 1:1 dès la récolte, les cellules de deux conditions expérimentales à comparer et ainsi limiter les biais techniques pouvant être introduits par les pertes inégales de protéines lors des étapes de lyse cellulaire et autres traitements des échantillons (enrichissement, dessalage, etc...).

La stratégie SILAC peut également être appliquée sur des animaux entiers et notamment des rongeurs. Ceux-ci sont nourris avec des aliments contenant des acides aminés isotopiquement lourds. Les tissus récupérés sur ces animaux contiennent donc des protéines marquées avec les isotopes lourds. Avantageusement, la méthode SILAC peut permettre l'incorporation d'isotope(s) présentant une différence de masse suffisante pour discriminer un peptide alourdi d'un peptide non alourdi. De plus, l'approche SILAC permet un marquage complet et stable de la totalité du protéome cellulaire assurant ainsi une quantification par spectrométrie de masse avec une très bonne reproductibilité et répétabilité.

Selon un mode de réalisation particulier de la présente invention, l'étape de fixation est réalisée avec un agent réticulant, par exemple le paraformaldéhyde, la formaline, le glutaraldéhyde.

Selon un mode de réalisation particulier de la présente invention, le compartiment subcellulaire est choisi dans le groupe constitué par le noyau, la membrane cytoplasmique, la membrane nucléaire, les vésicules, la mitochondrie, le lysosome, le centriole, le protéasome, les adhésions focales, le lamellipode, les filopodes, la rosette d'invadosomes, le réticulum endoplasmique, l'appareil de golgi, les jonctions cellule-cellule (*e.g.* synapses).

L'étape c) de marquage donc de repérage du compartiment subcellulaire d'intérêt peut être réalisée par différents moyens de marquage du compartiment subcellulaire tels que des peptides ou drogues. Selon un mode de réalisation particulier de la présente invention, l'étape de marquage du compartiment subcellulaire est réalisée avec un marqueur fluorescent. Ce marquage peut être réalisé après l'étape de fixation, ou avant par expression constitutive dans le compartiment subcellulaire de l'échantillon test d'un marqueur, typiquement fluorescent. Par exemple, il s'agit du peptide Lifeact ou de la phalloïdine pour l'actine filamenteuse des rosettes d'invadosomes, mais aussi du DAPI comme intercalant de l'ADN qui permet de visualiser les noyaux, de l'ER tracker pour le réticulum endoplasmique, etc....

L'étape e) d'extraction des protéines dudit compartiment subcellulaire test, de réversion de la fixation et/ou de protéolyse peuvent être réalisées par toutes techniques connues de l'homme du métier.

On entend par échantillon de référence de l'étape h), tout échantillon dont la concentration en peptides après comparaison avec la concentration en peptides dudit échantillon cellulaire test offre une indication de la capacité invasive d'une tumeur chez ledit sujet dont provient ledit échantillon cellulaire test.

À titre d'exemples d'échantillons de référence de l'étape h), on peut citer des échantillons cellulaires provenant d'un individu sain ou provenant d'un individu atteint d'une tumeur, ou encore une solution protéique à une concentration déterminée.

On entend par individu sain, un individu qui ne présente pas de pathologies, en particulier un individu qui ne présente pas de tumeur.

Est décrit un kit pour l'analyse protéomique subcellulaire d'un échantillon biologique test, notamment pour la mise en oeuvre du procédé d'analyse protéomique subcellulaire de l'invention, comprenant :
- au moins un moyen de marquage du compartiment subcellulaire test tel que défini ci-avant,
- au moins un moyen de réversion de la fixation et un moyen de réalisation de la protéolyse, et,
- au moins une solution protéique de référence à une concentration déterminée.

Le kit comprend en outre de façon préférée au moins un des moyens suivants, de préférence tous les moyens suivants :
- un moyen de marquage isotopique des protéines de l'échantillon test,
- un moyen de fixation des cellules dudit échantillon, et
- un moyen d'extraction des protéines dudit compartiment subcellulaire.

Le kit peut comprendre en outre tous les moyens nécessaires à la mise en oeuvre du procédé d'analyse protéomique subcellulaire de l'invention.

D'autres avantages pourront encore apparaître à l'homme du métier à la lecture des exemples ci-dessous, illustrés par les figures annexées, donnés à titre illustratif.

### Brève description des figures

- La figure 1 représente les grandes étapes d'un mode de réalisation du procédé de l'invention.

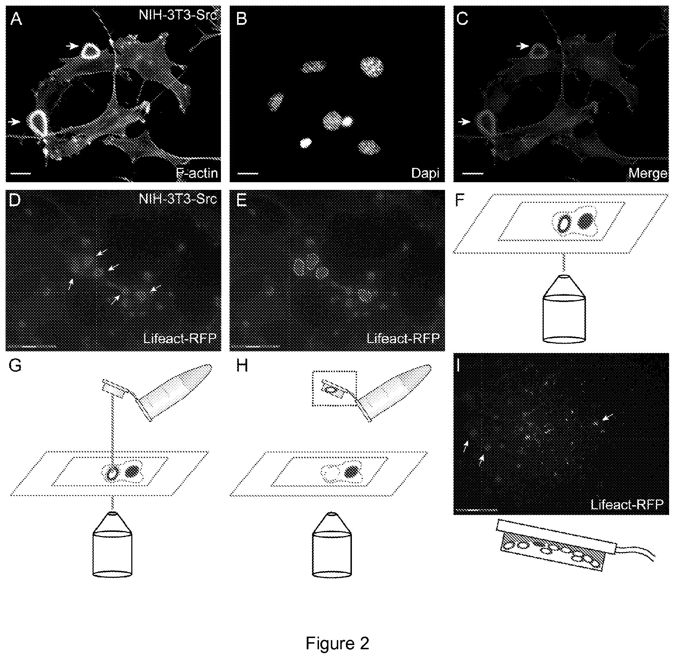

- La figure 2 représente (A-C) une image de microscopie à fluorescence de cellules NIH-3T3-Src marquées par le Dapi et la phalloïdine afin de révéler respectivement l'ADN du noyau et l'actine filamenteuse (les rosettes d'invadosomes sont indiquées par les flèches). (D-E). Ces images de microscopie montrent le même type cellulaire exprimant de manière stable le peptide Lifeact-RFP, les éléments microdisséqués sont entourés par des pointillés. (F) Ce schéma montre une rosette entourée manuellement par l'expérimentateur et découpée par un premier laser. (G-H) Ce schéma représente le processus de récupération des éléments microdisséqués par un second laser qui catapulte la rosette découpée au niveau du bouchon collecteur. (I) Cette image de microscopie montre la fluorescence des éléments microdisséqués au niveau du bouchon collecteur, le schéma associé représente ce même bouchon collecteur avec les éléments microdisséqués et récupérés.

- La figure 3 représente (A) la procédure d'automatisation de l'étape de microdissection laser, (B) une image représentative de microscopie à fluorescence montrant les rosettes qui ont été sélectionnées par le processus d'automatisation et qui ont été découpées par microdissection laser.

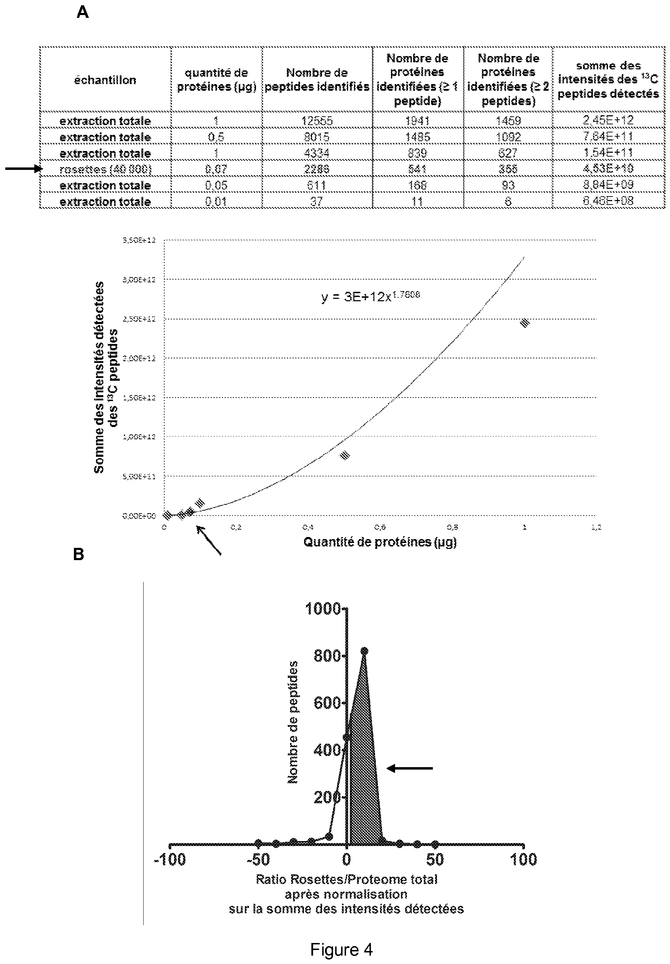

- La figure 4 représente (A) une analyse par spectrométrie de masse réalisée sur une gamme de quantité de protéines extraites de cellules NIH-3T3-Src totales marquées selon la méthode SILAC avec un isotope lourd (^{C13}R et ^{C13}K). A chaque quantité de protéines injectée correspond une somme des intensités de tous les peptides marqués détectés par le spectromètre de masse. La somme des intensités de tous les peptides marqués issus de 40000 rosettes découpées par microdissection laser a été comparée à cette gamme pour en déduire une quantité de 72ng de protéines extraites (Tableau, cf. ligne indiquée d'une flèche, et représentation graphique correspondante en-dessous indiquée d'une flèche). (B) La comparaison des intensités relatives des peptides identifiées dans l'échantillon de rosettes et l'échantillon lysat total (100ng) après normalisation sur la somme totale des intensités détectées en MS a permis de confirmer l'enrichissement des protéines identifiées dans les rosettes (ratio Rosettes/Total ≥2, aire grisé indiquée d'une flèche).

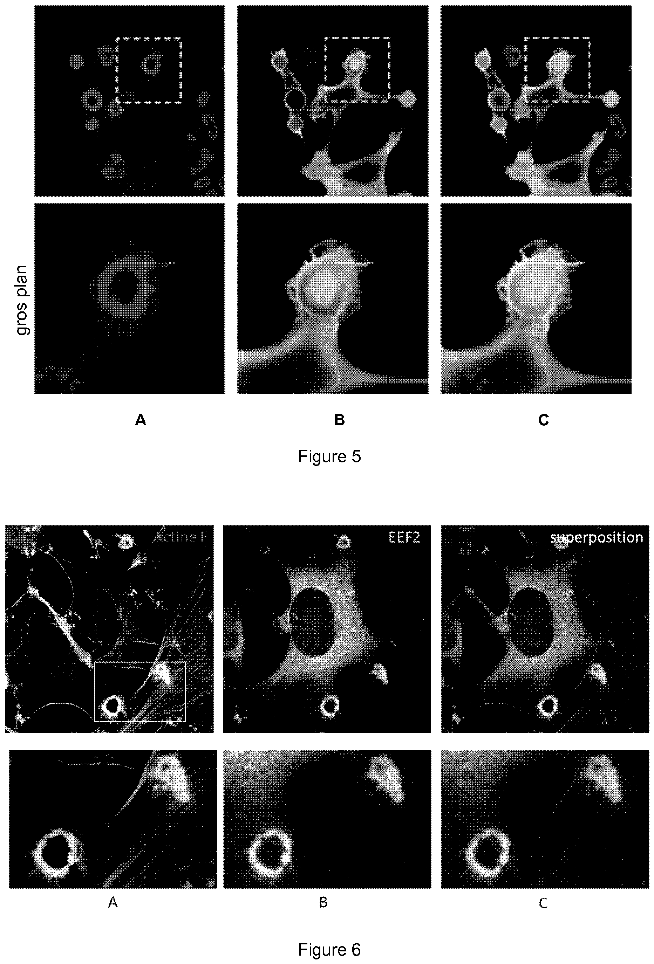

- La figure 5 représente une image de microscopie confocale de cellules NIH-3T3-src exprimant constitutivement le peptide life-Act et permettant de visualiser l'actine filamenteuse (A), ces cellules ont été transfectées avec un plasmide d'expression codant pour la protéine de fusion HA-eEF1A1, l'utilisation d'un anticorps anti-HA permettant de visualiser la localisation de cette protéine (B) la fusion de marquage de (A) et (B) sont représentés en (C). Les éléments encadrés sont représentés en gros plan sous chaque image, respectivement.
- La figure 6 représente une image de microscopie confocale de cellules NIH-3T3-src exprimant constitutivement le peptide life-Act et permettant de visualiser l'actine filamenteuse (A), ces cellules ont été transfectées avec un plasmide d'expression codant pour la protéine de fusion HA-Eif2A, l'utilisation d'un anticorps anti-HA permettant de visualiser la localisation de cette protéine (B). La fusion de marquage de (A) et (B) est représentée en (C). Les éléments encadrés sont représentés en gros plan sous chaque image, respectivement.

### EXEMPLES OU MODES DE REALISATION

### Exemple 1 : Analyse protéomique des rosettes d'invadosomes

Bien que le protocole qui suit soit adaptable à d'autres compartiments subcellulaires, le compartiment subcellulaire associé à la capacité des cellules à dégrader les éléments de la matrice extracellulaire a été choisi comme modèle pour la présente étude, à savoir la rosette d'invadosomes. Ce type de structure se forme dans des cellules exprimant de manière constitutive la forme active de l'oncogène c-Src et participe à la capacité d'invasion cellulaire. Il est crucial de connaître la composition protéique exacte et détaillée de ces structures afin de mettre en évidence des cibles thérapeutiques pour tenter d'inhiber l'invasion des cellules tumorales. L'actine filamenteuse est le composant majeur et structural de ces rosettes, et par conséquent l'élément qui a été choisi pour repérer ces rosettes au moment de la microdissection laser.

Les expérimentations ont été menées sur une lignée cellulaire générée à partir de cellules fibroblastiques NIH-3T3 qui expriment de manière constitutive l'oncogène c-Src (NIH3T3-src) et qui forment des rosettes d'invadosomes [7]. De plus, ces cellules expriment de manière constitutive le peptide de levure Lifeact [8] couplé à un fluorophore, à savoir le m-cherry (figure 2D). Ce peptide a la capacité de se lier spécifiquement à l'actine mais uniquement sous sa forme filamenteuse.

L'analyse par spectrométrie de masse d'une très faible quantité de matériel fait appel aux limites de sensibilité des spectromètres. Dans ce contexte une majorité des protéines identifiées sont en fait issues de contaminations provenant de la manipulation et de l'air ambiant. Pour discriminer les protéines des rosettes microdisséquées des éléments contaminants extérieurs (air ambiant, solutions, etc..), un marquage isotopique métabolique des protéines d'intérêt (C¹³Arginine (Arg⁶) et C¹³Lysine (Lys⁶)) a d'abord été réalisé selon la méthode SILAC (Stable Isotopic Labeling by Amino acids in Cell culture). Cette méthode consiste à utiliser un milieu de culture dépourvu en arginine et lysine dans lequel on ajoute de l'arginine et de la lysine marquées avec du carbone C¹³ et de la proline non marquée pour éviter la métabolisation de l'arginine marquée en proline marquée, et incorporer ce marquage pendant au moins 6 cycles de doublement cellulaire.

Les cellules exprimant le peptide lifeact couplé au fluorophore m-cherry (figure 2D) et marquées avec les isotopes lysine et arginine ont été cultivées sur un anneau membranaire en silicone (qu'on recouvre avec de gélatine afin de favoriser l'adhésion des cellules) placé dans une boite lumox dish 50 (Zeiss).

Après adhésion et rinçage avec du PBS, les cellules ont été fixées avec un agent réticulant, le paraformaldéhyde (PFA) à 4% dans une solution de PBS pendant 20 minutes. Après deux rinçages, les cellules ont été maintenues dans une solution de PBS à 4°C.

Ensuite ces cellules ont été marquées par le DAPI qui est un intercalant de l'ADN fluorescent qui permet de visualiser les noyaux.

Les cellules ont ensuite été placées sur un microdissecteur laser équipé d'un objectif X63 sec (Zeiss), et les cellules sont maintenues dans une fine pellicule de PBS.

Les rosettes à découper ont été entourées par l'intermédiaire d'un stylet, sur une tablette graphique (cercles pointillé) avant microdissection (figure 2E). Pour la découpe laser, un microscope Zeiss (PALM MicroBeam) a été utilisé.

Les rosettes ont ensuite été récoltées sur le bouchon d'un support (tube collecteur) en silicone (figure 2H).

Le support a ensuite été lavé avec une solution 50mM Tris-HCI pH 6,8, SDS 7,5%, glycérol 20%, bêta-mercaptoéthanol 5%, bleu de bromophénol 0,1% pendant 2 heures à 95°C, ce qui a permis d'extraire les protéines et de réverser leur fixation.

Cet extrait a ensuite été chargé dans un puits de gel SDS-PAGE 10% et mis sous une tension de 100 Volts jusqu'à migration du bleu de bromophénol jusqu'à la limite entre le gel de tassement et le gel de séparation en se servant d'un marqueur de masse moléculaire comme témoin visuel dans un autre puits.

Un carré de gel a ensuite été découpé entre la limite supérieure du puits et le front de migration a été traité pour réduction/alkylation des protéines puis protéolyse par la trypsine et extraction des peptides issus de cette digestion.

Les peptides ont ensuite été analysés par LC-MS/MS avec un gradient chromatographique C18 de 2 heures et une analyse sur un spectromètre de masse de type Q-Exactive (Thermo).

L'interrogation des banques de données a été réalisée avec deux algorithmes différents (Mascot et Sequest) en utilisant le logiciel Proteome Discoverer en incluant le marquage ^{C13}Arg et ^{C13}Lys comme modifications variables. Seuls les peptides de hauts scores, marqués et/ou identifiés avec l'un et/ou l'autre des deux algorithmes ont été conservés.

Le tableau 1 représente le bilan du nombre de protéines identifiées lors des différentes expériences. L'augmentation du nombre de pièces microdisséquées a d'abord permis d'augmenter le nombre de protéines identifiées pour atteindre 101 protéines identifiées après la découpe de 10 000 rosettes.

**Tableau 1**

| **Expérience** | **Nombre de pièces micro-disséquées** | **Nombre de protéines identifiées** | **Pourcentage de protéines strictement identiques par rapport à l'expérience précédente** |
|---|---|---|---|
| 1 | 350 | 9 | |
| 2 | 3000 | 55 | 44% |
| 3 | 10000 | 101 | 60% + 20% (protéines très proches) = 80% |

Il est également observé qu'en augmentant la quantité de matériel, plus de peptides sont identifiés, correspondant aux protéines identifiées lors de l'expérience précédente réalisée avec moins de matériel. Ainsi, entre l'expérience 2 et l'expérience 3, est confirmée l'identification de 60% des protéines et sont identifiées en plus 20% de protéines très similaires (isoformes, sous-unités différentes d'une même protéine) listées dans le tableau 2 suivant. Ces éléments démontrent la robustesse et la reproductibilité de cette technique.

**Tableau 2**

| **protéines de la 1ère expérience retrouvées dans les expériences 2 et 3** |
|---|
| Vimentin OS=Mus musculus GN=Vim |
| Actin, cytoplasmic 1 (Fragment) OS=Mus musculus GN=Actb |
| Histone H4 OS=Mus musculus GN=Hist1 h4a |
| Tubulin alpha-1C chain OS=Mus musculus GN=Tuba1c |

| **protéines communes entre les expériences 2 et 3** |
|---|
| Peroxiredoxin-1 (Fragment) OS=Mus musculus GN=Prdx1 |
| 40S ribosomal protein S3 OS=Mus musculus GN=Rps3 |
| ATP synthase subunit alpha OS=Mus musculus GN=Atp5a1 |
| Histone H3 (Fragment) OS=Mus musculus GN=H3f3a |
| Protein Ahnak OS=Mus musculus GN=Ahnak |
| Histone H2A OS=Mus musculus GN=Hist1h2al |
| Heterogeneous nuclear ribonucleoprotein H OS=Mus musculus GN=Hnrnph1 |
| Fructose-bisphosphate aldolase A OS=Mus musculus GN=Aldoa |
| Tubulin alpha-1B chain OS=Mus musculus GN=Tuba1b |
| Elongation factor 1-alpha 1 OS=Mus musculus GN=Eef1a1 |
| Histone H2B type 1-F/J/L OS=Mus musculus GN=Hist1 h2bf |
| Heat shock protein HSP 90-beta OS=Mus musculus GN=Hsp90ab1 |
| 40S ribosomal protein SA OS=Mus musculus GN=Rpsa |
| Glyceraldehyde-3-phosphate dehydrogenase OS=Mus musculus GN=Gapdh |
| Vimentin OS=Mus musculus GN=Vim |
| Stress-70 protein, mitochondrial OS=Mus musculus GN=Hspa9 |
| Isoform C of Prelamin-A/C OS=Mus musculus GN=Lmna |
| Heterogeneous nuclear ribonucleoprotein A1 OS=Mus musculus GN=Hnrnpa1 |
| Pyruvate kinase PKM OS=Mus musculus GN=Pkm |
| ATP synthase subunit beta, mitochondrial OS=Mus musculus GN=Atp5b |
| Elongation factor 2 OS=Mus musculus GN=Eef2 |
| Poly(rC)-binding protein 1 OS=Mus musculus GN=Pcbp1 |
| Actin, cytoplasmic 1 OS=Mus musculus GN=Actb |
| Histone H4 OS=Mus musculus GN=Hist1 h4a |
| Isoform 2 of 60 kDa heat shock protein, mitochondrial OS=Mus musculus GN=Hspd1 |
| Tubulin alpha-1A chain OS=Mus musculus GN=Tuba1a |
| Heat shock cognate 71 kDa protein OS=Mus musculus GN=Hspa8 |
| Polyubiquitin-B (Fragment) OS=Mus musculus GN=Ubb |
| Histone H2B type 2-E OS=Mus musculus GN=Hist2h2be |
| Cytoskeleton-associated protein 4 OS=Mus musculus GN=Ckap4 |
| Myosin-9 OS=Mus musculus GN=Myh9 |
| Nucleophosmin OS=Mus musculus GN=Npm1 |
| Phosphoglycerate kinase OS=Mus musculus GN=Pgk1 |

| **protéines de l'expérience 2 qui ressemblent beaucoup aux protéines détectées lors de l'expérience 3** |
|---|
| 60S ribosomal protein L31 OS=Mus musculus GN=Rpl31 |
| Elongation factor 1-delta (Fragment) OS=Mus musculus GN=Eef1d |
| T-complex protein 1 subunit gamma OS=Mus musculus GN=Cct3 |
| Heterogeneous nuclear ribonucleoprotein U, isoform CRA_b OS=Mus musculus GN=Gm28062 |
| Annexin A2 OS=Mus musculus GN=Anxa2 |
| 60S ribosomal protein L13 OS=Mus musculus GN=Rpl13 |
| Alpha-actinin-4 OS=Mus musculus GN=Actn4 |
| 40S ribosomal protein S8 OS=Mus musculus GN=Rps8 |
| Probable ATP-dependent RNA helicase DDX5 OS=Mus musculus GN=Ddx5 |
| Elongation factor 1-gamma OS=Mus musculus GN=Eef1g |
| 60S acidic ribosomal protein P0 (Fragment) OS=Mus musculus GN=Rplp0 |

### Exemple 2 : Validation de l'enrichissement

L'expérimentation a visé à démontrer que le procédé de l'invention (marquage isotopique + microdissection laser ciblée + spectrométrie de masse) permet d'enrichir l'échantillon avec les protéines spécifiquement exprimées par le compartiment subcellulaire d'intérêt (*i.e.* les rosettes).

Une gamme de quantité de protéines d'un lysat cellulaire total a été utilisée comme point de comparaison, pour atteindre la même quantité que celle récoltée avec les 40000 rosettes, et pouvoir servir de référence (72ng).

Les résultats sont présentés dans la figure 4.

### Exemple 3 : Validation par marquage de la présence des protéines identifiées (e.g. facteur d'élongation 1-α1)

Les cellules NIH3T3-src ont été ensemencées sur lamelle de verre puis transfectées avec le plasmide HA-eEF1A1 (donné par le Dr. IRWIN MS, University of Toronto, Ontario, Canada) par l'utilisation d'un agent de transfection, la lipofectamine 2000 (thermo-Fisher), selon le protocole décrit par le fabricant. Ensuite les cellules ont été rincées après 6 heures d'incubation, et laissées au repos pendant 48h. Puis, les cellules ont été fixées par l'ajout d'une solution de paraformaldéhyde 4%-PBS pendant 10 minutes.

Puis il a été procédé au protocole d'immunofluorescence, les cellules ont été perméabilisées par une solution de Triton. Enfin, les cellules ont été incubées avec l'anticorps primaire anti-HA (3F10, Roche) dans une solution anticorps primaire-PBS-BSA, puis après lavages avec une solution contenant un anticorps secondaire couplé à un fluorophore avant observation avec un microscope confocal (Leica SP5).

Les résultats sont présentés dans la figure 5.

### Exemple 4 : Validation par marquage de la présence des protéines identifiées (e.g. HA-Eif2A)

Les cellules NIH3T3-src ont été ensemencées sur lamelle de verre puis transfectées avec le plasmide Flag-EEF2, (donné par le Dr. Huang YS, Academia Sinica, Taipei, Taiwan) par l'utilisation d'un agent de transfection, la lipofectamine 2000 (thermo-Fisher), selon le protocole décrit par le fabricant. Ensuite les cellules ont été rincées après 6 heures d'incubation, et laissées au repos pendant 48h. Puis, les cellules ont été fixées par l'ajout d'une solution de paraformaldéhyde 4%-PBS pendant 10 minutes.

Puis il a été procédé au protocole d'immunofluorescence, les cellules ont été perméabilisées par une solution de Triton. Enfin, les cellules ont été marquées par un anticorps anti-flag (Sigma F1804) dans une solution anticorps anti-flag-PBS-BSA, puis après lavages avec une solution contenant un anticorps secondaire couplé à un fluorophore avant observation avec un microscope confocal (Leica SP5).

Les résultats sont présentés dans la figure 6.

Ces expériences, décrites dans les exemples 3 et 4, ont permis de mettre en évidence la présence de ces protéines, eEF1A1 et HA-Eif2A, au niveau des rosettes et ainsi de valider la procédure d'identification de nouveaux marqueurs au niveau des rosettes.

De manière similaire, des expériences ont été faites et valident la présence des protéines EEF2, EIF3H, eif4E et Reptin au niveau des rosettes.

### Listes des références

1. Drissi, R., M.L. Dubois, and F.M. Boisvert, Proteomics methods for subcellular proteome analysis. FEBS J, 2013. 280(22): p. 5626-34.
2. Havrylov, S. and M. Park, MS/MS-based strategies for proteomic profiling of invasive cell structures. Proteomics, 2015. 15(2-3): p. 272-86.
3. Gessel, M.M., J.L. Norris, and R.M. Caprioli, MALDI imaging mass spectrometry: spatial molecular analysis to enable a new age of discovery. J Proteomics, 2014. 107: p. 71-82.
4. Gregorich, Z.R. and Y. Ge, Top-down proteomics in health and disease: challenges and opportunities. Proteomics, 2014. 14(10): p. 1195-210.
5. Ye, H., et al., Top-down proteomics with mass spectrometry imaging: a pilot study towards discovery of biomarkers for neurodevelopmental disorders. PLoS One, 2014. 9(4): p. e92831.
6. Ait-Belkacem, R., et al., MALDI imaging and in-source decay for top-down characterization of glioblastoma. Proteomics, 2014. 14(10): p. 1290-301.
7. Abram, C.L., et al., The adaptor protein fish associates with members of the ADAMs family and localizes to podosomes of Src-transformed cells. J Biol Chem, 2003. 278(19): p. 16844-51.
8. Riedl, J., et al., Lifeact: a versatile marker to visualize F-actin. Nat Methods, 2008. 5(7): p. 605-7.
9. Demande de Brevet EP1601450.
10. Leverenz et al., Proteomic Identification of Novel Proteins in Cortical Lewy Bodies. Brain Pathol., 2007. 17(2): p. 139-145.
11. Demande de Brevet US2011/0215233.

## Revendications

1. Procédé d'analyse protéomique subcellulaire d'un échantillon biologique test comprenant les étapes suivantes :
a) Marquage isotopique métabolique des protéines d'un échantillon biologique test ;
b) Fixation dudit échantillon ;
c) Marquage du compartiment subcellulaire test ;
d) Microdissection laser dudit compartiment subcellulaire ;
e) Extraction des protéines dudit compartiment subcellulaire, réversion de la fixation et protéolyse ;
f) Analyse des peptides issus de l'étape e) par spectrométrie de masse ;
g) Identification des peptides analysés.

2. Procédé selon la revendication 1, où l'étape de fixation est réalisée avec un agent réticulant.

3. Procédé selon la revendication 2, où l'agent réticulant est le paraformaldéhyde.

4. Procédé selon la revendication 1, où l'étape de marquage du compartiment subcellulaire est réalisée avec un marqueur fluorescent.

5. Procédé selon l'une quelconque des revendications 1 à 4, dans lequel le compartiment subcellulaire est choisi dans le groupe constitué par le noyau, la membrane cytoplasmique, la membrane nucléaire, les vésicules, la mitochondrie, le lysosome, le centriole, le protéasome, les adhésions focales, le lamellipode, les filopodes, la rosette d'invadosomes, le réticulum endoplasmique, l'appareil de golgi, les jonctions cellule-cellule.

6. Procédé d'identification *in vitro* de peptides correspondant à une protéine présente dans un compartiment subcellulaire d'un échantillon biologique d'un sujet comprenant les étapes suivantes :
a) Marquage isotopique métabolique des protéines d'un échantillon biologique d'un sujet ;
b) Fixation dudit échantillon ;
c) Marquage dudit compartiment subcellulaire test ;
d) Microdissection laser dudit compartiment subcellulaire ;
e) Extraction des protéines dudit compartiment subcellulaire, réversion de la fixation et protéolyse ;
f) Analyse des peptides issus de l'étape e) par spectrométrie de masse ;
g) Identification des peptides analysés ;
h) Comparaison qualitative et/ou quantitative des peptides issus de l'étape g) par rapport aux peptides présents dans un échantillon de référence ou à une valeur de référence.

7. Procédé selon la revendication 6, où ledit compartiment subcellulaire est la rosette d'invadosome.

8. Procédé selon l'une quelconque des revendications 1 à 7, où l'étape de marquage isotopique est réalisée par la méthode SILAC.

## Patentansprüche

1. Subzelluläres Proteomanalyseverfahren einer biologischen Prüfprobe, umfassend die folgenden Schritte:
a) metabolische Isotopenmarkierung der Proteine einer biologischen Prüfprobe;
b) Fixierung der Probe;
c) Markierung des subzellulären Prüfkompartiments;
d) Laser-Mikrodissektion des subzellulären Kompartiments;
e) Extraktion der Proteine des subzellulären Kompartiments, Umkehrung der Fixierung und Proteolyse;
f) Analyse der aus Schritt e) stammenden Peptide durch Massenspektrometrie;
g) Identifikation der analysierten Peptide.

2. Verfahren nach Anspruch 1, wobei der Fixierungsschritt mit einem Vernetzungsmittel durchgeführt wird.

3. Verfahren nach Anspruch 2, wobei das Vernetzungsmittel Paraformaldehyd ist.

4. Verfahren nach Anspruch 1, wobei der Markierungsschritt des subzellulären Kompartiments mit einem fluoreszierenden Marker durchgeführt wird.

5. Verfahren nach einem der Ansprüche 1 bis 4, wobei das subzelluläre Kompartiment gewählt wird aus der Gruppe, bestehend aus dem Kern, der zytoplasmatischen Membran, der Kernmembran, den Vesikeln, dem Mitochondrium, dem Lysosom, dem Zentriol, dem Proteasom, den fokalen Adhäsionen, dem Lamellipodium, den Filopodia, der Invadosomrosette, dem endoplasmatischen Retikulum, dem Golgiapparat, den Zelle-Zelle-Verbindungsstellen.

6. *In vitro*-Identifikationsverfahren von Peptiden, die einem in einem subzellulären Kompartiment einer biologischen Probe eines Subjekts vorhandenem Protein entsprechen, umfassend die folgenden Schritte:
a) metabolische Isotopenmarkierung der Proteine einer biologischen Probe eines Subjekts;
b) Fixierung der Probe;
c) Markierung des subzellulären Prüfkompartiments;
d) Laser-Mikrodissektion des subzellulären Kompartiments;
e) Extraktion der Proteine des subzellulären Kompartiments, Umkehrung der Fixierung und Proteolyse;
f) Analyse der aus Schritt e) stammenden Peptide durch Massenspektrometrie;
g) Identifikation der analysierten Peptide;
h) qualitativer und/oder quantitativer Vergleich der aus Schritt g) stammenden Peptide in Bezug auf die in einer Referenzprobe oder bei einem Referenzwert vorhandenen Peptide.

7. Verfahren nach Anspruch 6, wobei das subzelluläre Kompartiment die Invadosomrosette ist.

8. Verfahren nach einem der Ansprüche 1 bis 7, wobei der Schritt der Isotopenmarkierung durch das SILAC-Verfahren durchgeführt wird.

## Claims

1. A process for the subcellular proteomic analysis of a test biological sample, comprising the following steps:
a) metabolic isotopic labeling of the proteins from a test biological sample;
b) fixation of said sample;
c) labeling of the test subcellular compartment;
d) laser microdissection of said subcellular compartment;
e) extraction of the proteins from said subcellular compartment, reversion of the fixation, and proteolysis;
f) analysis of the peptides resulting from step e) by mass spectrometry;
g) identification of the peptides analyzed.

2. The process as claimed in claim 1, wherein the fixation step is performed with a crosslinking agent.

3. The process as claimed in claim 2, wherein the crosslinking agent is paraformaldehyde.

4. The process as claimed in claim 1, wherein the step of labeling the subcellular compartment is performed with a fluorescent marker.

5. The process as claimed in any one of claims 1 to 4, wherein the subcellular compartment is chosen from the group consisting of the nucleus, cytoplasmic membrane, nuclear membrane, vesicles, mitochondria, lysosome, centriole, proteasome, focal adhesions, lamellipodium, filopodia, invadosome rosette, endoplasmic reticulum, Golgi apparatus, and cell-cell junctions.

6. A process for the *in vitro* identification of peptides corresponding to a protein being present in the subcellular compartment of a biological sample from a subject, comprising the following steps:
a) metabolic isotopic labeling of the proteins from a biological sample from a subject;
b) fixation of said sample;
c) labeling of said test subcellular compartment;
d) laser microdissection of said subcellular compartment;
e) extraction of the proteins from said subcellular compartment, reversion of the fixation, and proteolysis;
f) analysis of the peptides resulting from step e) by mass spectrometry;
g) identification of the peptides analyzed;
h) qualitative and/or quantitative comparison of the peptides resulting from step g) relative to the peptides present in a reference sample or to a reference value.

7. The process as claimed in claim 6, wherein said subcellular compartment is the invadosome rosette.

8. The process as claimed in any one of claims 1 to 7, wherein the isotope labeling step is performed by the SILAC method.
